(19) Europäisches Patentamt
European Patent Office
Office européen des brevets

(11) EP 0 750 656 B1

(12) EUROPÄISCHE PATENTSCHRIFT

(45) Veröffentlichungstag und Bekanntmachung des Hinweises auf die Patenterteilung:
**29.12.1997 Patentblatt 1997/52**

(51) Int Cl.[6]: **C09K 19/34**, C09K 19/40, C09K 19/58, C07D 493/04, C07F 5/02

(21) Anmeldenummer: **95912229.2**

(22) Anmeldetag: **09.03.1995**

(86) Internationale Anmeldenummer:
**PCT/EP95/00866**

(87) Internationale Veröffentlichungsnummer:
**WO 95/25150 (21.09.1995 Gazette 1995/40)**

# (54) CHIRALE VERBINDUNGEN

CHIRAL COMPOUNDS

COMPOSES CHIRAUX

(84) Benannte Vertragsstaaten:
**CH DE FR GB LI NL**

(30) Priorität: **15.03.1994 DE 4408804**

(43) Veröffentlichungstag der Anmeldung:
**02.01.1997 Patentblatt 1997/01**

(73) Patentinhaber: **BASF AKTIENGESELLSCHAFT**
**67056 Ludwigshafen (DE)**

(72) Erfinder:
- **SIEMENSMEYER, Karl**
**D-67227 Frankenthal (DE)**
- **VILL, Volkmar**
**D-20255 Hamburg (DE)**
- **TUNGER, Hanns-Walter**
**D-22085 Hamburg (DE)**

(56) Entgegenhaltungen:
**DE-A- 4 200 819** **GB-A- 2 165 541**

- **ZEITSCHRIFT FUR NATURFORSCHUNG, TEIL B: ANORGANISCHE CHEMIE, ORGANISCHE CHEMIE, Bd. 41b, Nr. 6, Juni 1986 TÜBINGEN DE, Seiten 736-750, B. KOHNE ET AL. 'calamitische flüssigkristalle: Beziehung zwischen molekülstruktur und mesogenität, trans- und cis-1,3,5,7-tetraoxadecalin-derivate'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 750 656 B1

## Beschreibung

Chirale, smektisch flüssigkristalline Materialien, die beim Abkühlen aus der flüssigkristallinen Phase glasartig unter Ausbildung einer Schichtstruktur erstarren, werden bekanntermaßen auf elektrooptischem Gebiet für viele Zwecke eingesetzt. Zu nennen sind hier beispielsweise optische Speichersysteme (DE-A-38 27 603 und DE-A-39 17 196), die Elektrophotografie (DE-A-39 30 667), flüssigkristalline Anzeigeelemente wie Displays (Mol. Cryst. Liq. Cryst., 114, 151 (1990)) sowie bei gleichzeitig vorliegendem ferroelektrischem Verhalten elektrische Speichersysteme (Ferroelectrics, 104, 241(1990)).

In der Schichtstruktur ferroelektrischer $S_c$*-Phasen sind die Moleküllängsachsen innerhalb der einzelnen Schicht gegenüber der Schichtnormalen z geneigt. Die Richtung dieser Neigung wird durch den Direktor n angegeben, der Winkel zwischen z und n ist der sogenannte Tiltwinkel Θ. $S_c$*-Phasen weisen zwei stabile Zustände mit unterschiedlicher Richtung von n auf, zwischen denen durch Anlegen eines elektrischen Feldes geschaltet werden kann (elektrooptischer Effekt).

$S_c$*-Phasen treten bei niedermolekularen, flüssigkristallinen Materialien, bei Oligomesogenen und bei polymer ferrolelektrischen Materialien auf, wobei die wesentlichen Eigenschaften der $S_c$*-Phasen übereinstimmen.

Die bislang hergestellten flüssigkristallinen Materialien weisen jedoch Nachteile auf, zum Beispiel geringe spontane Polarisation, geringe Phasenbreite, kein stabiles, getiltet smektisches Glas bei Raumtemperatur oder zu langsames Schalten.

Das Auftreten der flüssigkristallinen $S_c$*-Phase wird durch alle Gruppen des Moleküls, d.h. Seitengruppe A, mesogene Gruppe M und die chirale Gruppe in erheblichem Ausmaß beeinflußt, so daß kleinste Änderungen der molekularen Struktur $S_c$*-Phasen induzieren oder auch zum Verschwinden bringen können.

Speziell die chirale Gruppe ist durch ihre Struktur und spezielle Funktion für das Zustandekommen einer spontanen Polarisation von entscheidender Bedeutung.

Der vorliegenden Erfindung liegt die Aufgabe zugrunde, chirale Gruppen für flüssigkristalline Materialien zu suchen, die die flüssigkristallinen Eigenschaften möglichst wenig stören, gleichzeitig hohe spontane Polarisationen induzieren und synthetisch verfügbar sind.

Die Erfindung betrifft daher Verbindungen der allgemeinen Formel I

$$A\text{-}Y\text{-}(M\text{-}Y)_n\text{-}Z\text{-}(Y\text{-}M)_n\text{-}Y\text{-}A \qquad I$$

in der

Z ein Rest der Formel

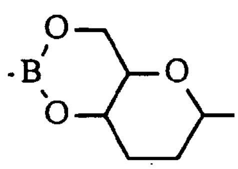

oder

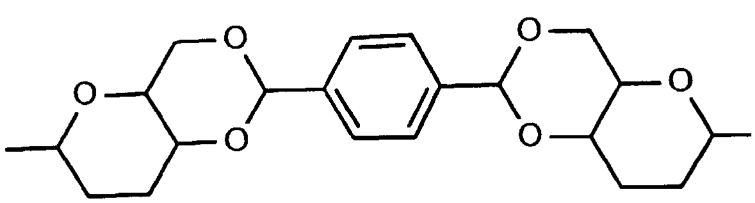

ist und wobei die Reste

A unabhängig voneinander gegebenenfalls durch Fluor, Chlor, Brom oder Cyan substituiertes und gegebenenfalls

durch O, S, NH, $NCH_3$, COO oder OCO unterbrochenes $C_1$- bis $C_{30}$-Alkyl,

Y unabhängig voneinander eine chemische Bindung, O, S, NH, $N(CH_3)$, COO oder OCO und

M unabhängig voneinander eine mesogene Gruppe aus einem aromatischen oder aliphatischen Ringsystem der allgemeinen Formel II

$$-Y^1-(Z^1-Y^1)_m- \qquad \text{II}$$

bedeuten, in dem die Reste

$Y^1$ unabhängig voneinander eine direkte Bindung, O, COO, OCO, $CH_2O$, $OCH_2$, CH=N oder N=CH,

$Z^1$ unabhängig voneinander gegebenenfalls durch Fluor, Chlor, Brom, Jod, Cyan, Hydroxy, Methoxy oder Methyl substituiertes p-Phenylen, 1,3,4-Thiadiazolylen-2,5 oder Pyrimidylen-2,5 und

m eine ganze Zahl zwischen 1 und 3 bedeuten, und

n unabhängig voneinander 0 oder 1 ist mit der Maßgabe, daß ein n von 0 verschieden ist.

Insbesondere betrifft die Erfindung Verbindungen der allgemeinen Formel I, bei denen die Reste

A unabhängig voneinander gegebenenfalls durch O, COO oder OCO unterbrochenes $C_1$ bis $C_{12}$-Alkyl, und

Y unabhängig voneinander eine direkte Bindung oder O, COO oder OCO sind.

Die Verwendung der erfindungsgemäßen Verbindungen als Dotierstoff in flüssigkristallinen Medien, zum Aufbau von Aufzeichnungsschichten für laseroptische und elektrische Aufzeichnungselemente, in der Elektrophotografie, zur Erzeugung latenter Ladungsbilder, zum Aufbau oder als Bestandteil von flüssigkristallinen Anzeigeelementen sowie als farbige Reflektoren ist ebenfalls Gegenstand der Erfindung.

Der mesogene Molekülteil M besteht aus einem aromatischen oder aliphatischen Ringsystem der allgemeinen Formel II

$$-Y^1-(Z^1-Y^1)_m- \qquad \text{II}$$

in dem die Reste

$Y^1$ unabhängig voneinander eine direkte Bindung, O, COO, OCO, $CH_2O$, $OCH_2$, CH=N oder N=CH,

$Z^1$ unabhängig voneinander gegebenenfalls durch Fluor, Chlor, Brom, Jod, Cyan, Hydroxy, Methoxy oder Methyl substituiertes p-Phenylen, 1,3,4-Thiadiazolylen-2,5 oder Pyrimidylen-2,5 und

m eine ganze Zahl zwischen 1 und 3 bedeuten.

Beispiele für besonders bevorzugte Gruppen $Z^1$ sind

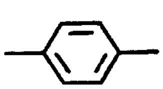 , 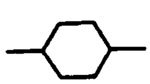 , 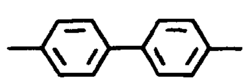 , 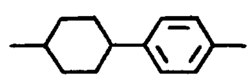 ,

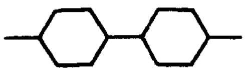 , 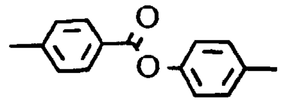 , 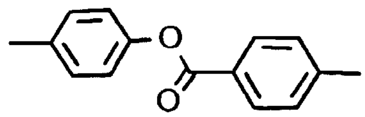 ,

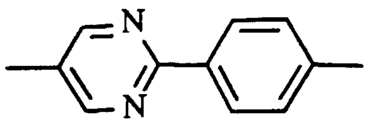 , 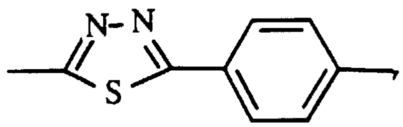 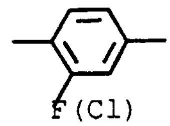 ,

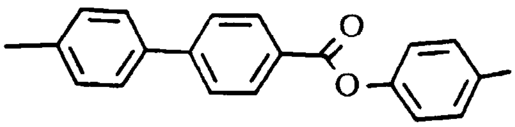 , 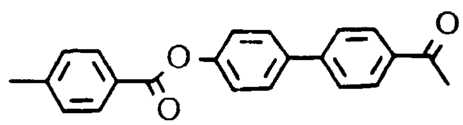

Die erfindungsgemäßen Einheiten A-Y-$(M-Y)_n$, in denen A, Y und M die oben angegebene Bedeutung besitzen, sind im Prinzip durch allgemeinbekannte Syntheseverfahren, wie sie z.B. in der DE-A-39 17 186 und in Mol.Cryst.Liq. Cryst.191,231(1991) beschrieben sind zugänglich.

Alle Reaktionen wurden dünnschichtchromatographisch (DC) auf Kieselgel-Fertigfolie GF 245 (Merck) verfolgt. Die Detektion erfolgte durch UV-Fluoreszenzlöschung und/oder Besprühen mit 15 %iger ethanolischer Schwefelsäure und nachfolgende Wärmbehandlung.

Säulenchromatographische Trennungen wurden durch Flash-Chromatographie an Kieselgel 60 (230-400 mesh, Merck) durchgeführt.

Die Messungen der optischen Drehungen wurden mit einem Perkin-Elmer-Polarimeter 589 (Natrium-D-Linie) durchgeführt.

Die NMR-Spektren ($^{1}$H: 400 MHz, $^{13}$C: 100,67 MHz) wurden im Servicebetrieb an einem Bruker AMX-400 NMR-Spektrometer gemessen. Die Auswertung erfolgte nach erster Ordnung.

Die Phasenumwandlungstemperaturen wurden mit einem Leitz Polarisationsmikroskop (Ortholux II pol) in Verbindung mit einem Mettler Mikroskopheiztisch (Mettler FP 800/84) bestimmt. Die folgenden Abkürzung wurden zur Bezeichnung der Phasen durchgängig verwendet (chirale Phasen sind mit * gekennzeichnet):

K kristalline Phase,
$S_C$ smektische C Phase
$S_A$ smektische A Phase
Ch cholesterische Phase
I isotrop flüssige Phase
D kubische D-Phase
Q smektische Q-Phase

Allgemeine Arbeitsvorschriften:

Darstellung der Phenylether (Vorschrift 1)

25 mmol des Phenols werden in 15 ml Aceton gelöst, mit 50 mmol des Alkylhalogenids sowie 30 mmol (4,14 g) Kaliumcarbonat versetzt und unter Rückfluß gekocht, bis das gesamte Phenol umgesetzt ist. Das Aceton wird am Rotationsverdampfer abgezogen und der Rückstand im Vakuum destilliert.

Umsetzung der C-Glycoside mit Boronsäuren (Vorschrift 2)

10 mmol β-Glycosid werden in 4 ml Toluol aufgenommen und mit 20 mmol der Boronsäure versetzt. Durch mehrmaliges Abziehen des Lösungsmittels am Rotationsverdampfer im Vakuum wird das bei der Reaktion entstehende Wasser mit dem Toluol azeotrop abdestilliert. Der kristalline Ruckstand wird aus Ethanol umkristallisiert.

Umacetalisierung von Dimethylacetalen mit C-Glycosiden (Vorschrift 3)

8 mmol des β-Glycosides werden mit 9,6 mmol des Dimethylacetales und einer Spatelspitze p-Toluolsulfonsäure in 4 ml absoluten Dimethylformamid gelöst und am Rotationsverdampfer bei einer Wasserbadtemperatur von 65 - 69°C sowie einem Druck von 29 - 33 hPa umgesetzt. Nach Reaktionsende wird das Lösungsmittel abgezogen und der Rückstand nach der Auskristallisation mit einigen ml gesättigter Natriumhydrogencarbonatlösung versetzt, abfiltriert, mit Wasser und kaltem Ethanol gewaschen und aus Ethanol umkristallisiert.

Beispiel 1

Herstellung von (1S,3R,6R,8R)-3-(4''-heptyloxyphenyl)-8-(4'-decyloxyphenyl)-3-bora-2,4,7-trioxybicyclo-[4,4,O]-decan

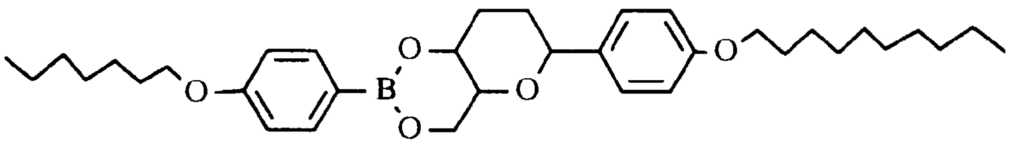

a) Herstellung von 1'-(4,6-Di-O-acetyl-2,3-didesoxy-β-D-erythrohexopyranosyl)-4'-decyloxybenzol

2 g Tri-O-acetyl-D-glucal und 4 g Decyloxybenzol werden in 50 ml trockenem Dichlormethan mit 2 Tropfen Zinntetrachlorid versetzt und zwei Stunden bei Raumtemperatur gerührt. Nach Zugabe von 2 g festem Natriumcarbonat und 15 min Rühren wird filtriert, das Dichlormethan im Vakuum entfernt und das Produktgemisch in 40 ml Ethanol und 120 ml Ethylacetat aufgenommen. Es wird unter Zusatz von 10 mg Palladium auf Aktivkohle (10%) unter Wasserstoffatmosphäre vier Stunden bei Raumtemperatur gerührt, filtriert und im Vakuum eingeengt. Die Reinigung erfolgt durch Säulenchromatografie (Kieselgel 60, Korngröße 0.04-0.063, Laufmittel: Petrolether/Ethylacetat 20/1).

Ausbeute: 28 %

Schmelzpunkt: 53.4°C

b) Herstellung von 1'-(2,3-Didesoxy-β-D-erythro-hexopyranosyl)-4'-decyloxybenzol

Zur Deacetylierung des Produktes aus la wird dieses in 50 ml Methanol aufgenommen und mit 10 mg Natriummethanolat versetzt. Nach 4 stündigem Rühren bei Raumtemperatur wird mit saurem Ionenaustauscher (Amberlite IR 120 $H^+$-Form neutralisiert, filtriert und das Lösungsmittel im Vakuum entfernt.

Schmelzpunkt: 103-106°C

c) 50 mg 1'-(2,3-Didesoxy-β-D-erythro-hexopyranosyl)-4'-decyloxybenzol werden mit 25 mg 4-Heptyloxyphenylboronsäure in 5 ml Toluol aufgenommen. Im Vakuum wird am Rotationsverdampfer das entstehende Wasser mit Toluol azeotrop abdestilliert. Der Vorgang wird dreimal wiederholt. Der kristalline Rückstand wird aus Ethanol umkristallisiert.

Ausbeute: 57%

Phasenverhalten: K 76.8 ($S_C$ 57) $S_A$ 173-174 I

Beispiel 2

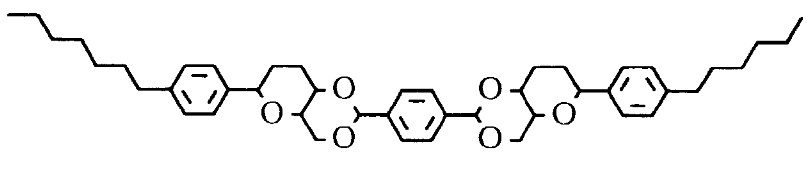

a) Herstellung von 1'-(4,6-Di-O-acetyl-2,3-didesoxy-β-D-erythrohexopyranosyl)-4'-hexylbenzol und -4'heptylbenzol.

Die Herstellung der Verbindungen erfolgte analog zu Beispiel 1a) unter Einsatz von Hexylbenzol bzw. Heptylbenzol anstelle von Decyloxybenzol.

b) Herstellung von 1'-(2,3-didesoxy-β-D-erythro-hexopyranosyl)-4'-hexylbenzol und -4'heptylbenzol

Die Deacetylierung der Produkte aus 2a) erfolgte analog zu Beispiel 1b).

c) Die Herstellung der Titelverbindung erfolgte durch Umsetzung der Verbindungen aus 2b) mit dem Tetramethylacetat des Terephthalaldehyds gemäß Vorschrift 3 unter Berücksichtigung des Molverhältnisses.

Phasenverhalten: K 192 $S_C$ 233 Ch >300 Zersetzung

Die Verbindungen der Beispiele 3 bis Beispiel 24 wurden analog Beispiel 1 hergestellt.

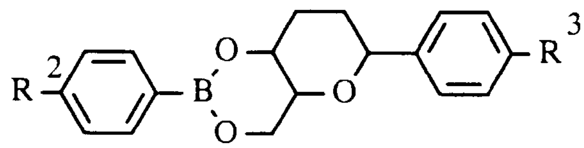

| Beispiel | $R^2$ | $R^3$ | Phasenverhalten |
|---|---|---|---|
| 3 | $CH_3O$- | $-OCH_3$ | K 158 Ch 211 I |
| 4 | $CH_3O$- | $-OC_6H_{13}$ | K 102 Ch 182 |
| 5 | $CH_3O$- | $-OC_8H_{17}$ | K 95 Ch 165 I |
| 6 | $CH_3O$- | $-OC_{10}H_{21}$ | K 85 Ch 156 I |
| 7 | $CH_3O$- | $-OC_{12}H_{25}$ | K 94 $S_A$ 103 Ch 149 I |
| 8 | $C_6H_{13}O$- | $-OCH_3$ | K 92 Ch 176 I |

| Beispiel | $R^2$ | $R^3$ | Phasenverhalten |
|---|---|---|---|
| 9 | $C_6H_{13}O$- | $-OC_6H_{13}$ | K 94 $S_A$ 180 I |
| 10 | $C_6H_{13}O$- | $-OC_8H_{17}$ | K 83 $S_A$ 180 Ch >195 Zersetzung |
| 11 | $C_6H_{13}O$- | $-OC_{10}H_{21}$ | K 76 ($S_C$ 57) $S_A$ 177 I |
| 12 | $C_6H_{13}O$- | $-OC_{12}H_{25}$ | K 82 ($S_C$ 44) $S_A$ 168 I |
| 13 | $C_8H_{17}O$- | $-OCH_3$ | K 90 $S_A$ 170 I |
| 14 | $C_8H_{17}O$- | $-OC_8H_{17}$ | K 85 $S_A$ 178 I |
| 15 | $C_8H_{17}O$- | $-OC_{10}H_{21}$ | K 83 $S_A$171 I |
| 16 | $C_8H_{17}O$- | $-OC_{12}H_{25}$ | K 85 ($S_C$ 52) $S_A$ 178 I |
| 17 | $C_{10}H_{21}O$- | $-OCH_3$ | K 93 Ch 163 I |
| 18 | $C_{10}H_{21}O$- | $-OC_8H_{17}$ | K 87 $S_A$ 172 I |
| 19 | $C_{10}H_{21}O$- | $-OC_{10}H_{21}$ | K 93 $S_A$ 167 I |
| 20 | $C_{10}H_{21}O$- | $-OC_{12}H_{25}$ | K 89 $S_A$ 162 I |
| 21 | $C_{12}H_{25}O$- | $-OCH_3$ | K 96 Ch 151 I |
| 22 | $C_{12}H_{25}O$- | $-OC_8H_{17}$ | K 89 $S_A$ 165 I |
| 23 | $C_{12}H_{25}O$- | $-OC_{10}H_{21}$ | K 90 $S_A$ 160 I |
| 24 | $C_{12}H_{25}O$- | $-OC_{12}H_{25}$ | K 95 $S_A$ 156 I |

Beispiel 25

(1S,6R,8R)-3-(4'-octoxyphenyl)-8-(2"-fluor-4"-hexoxyphenyl)-2,4,7-trioxa-3-borabicyclo[4.4.0.]decan

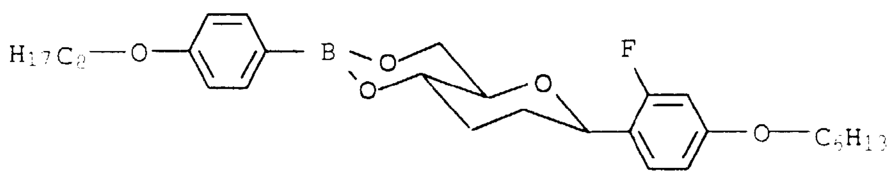

Synthese des C-Glycosides 1'-12,3-Didesoxy-β-D-erythro-hexopyranosyl)-2'-fluor-4'-hexoxybenzol

Zu einer Lösung von 11 mmol (3,06 g) Tri-O-acetyl-D-glucal in 50 ml absolutem Dichlormethan werden 3,31 g (16,87 mmol) 3-Fluorhexoxybenzol gegeben, mit 2 Tropfen Zinntetrachlorid versetzt und bei Raumtemperatur gerührt. Die Zugabe von Zinntetrachlorid kann dabei je nach Umsatz nach einiger Zeit wiederholt werden. Nach Ablauf der Reaktion werden 2 g festes Natriumcarbonat zugegeben, nach 15 weiteren Minuten Rühren wird abfiltriert und das Lösungsmittel am Rotationsverdampfer abgezogen.

Der Rückstand wird in Ethanol/Ethylacetat 1:3 aufgenommen, mit 10 mg Palladium auf Kohle versetzt und unter einer Wasserstoffatmosphäre bei Raumtemperatur gerührt. Nach Reaktionsende wird vom Katalysator abfiltriert und das Lösungsmittel am Rotationsverdampfer entfernt. Das Produktgemisch wird durch Säulenchromatogrpahie (Laufmittel Petrolether 60/70:Essigester 20:1) aufgetrennt.

Zur Deacetylierung wird das Produkt in 50 ml Methanol gelöst, mit einer Spatelspitze Natriummethanolat versetzt und über Nacht bei Raumtemperatur gerührt. Nach Zugabe eines sauren Ionenaustauschers (Amberlite ® IR 120, $H^+$-Form) zur Neutralisation wird der Austauscher abfiltriert und das Methanol im Vakuum entfernt.
Ausbeute: 0,219 g, $[\alpha]_D^{20}$ = +19,0 (c = 0,5, $CHCl_3$)

Die Darstellung der Titelverbindung erfolgt durch Umsatz von 35 mg (0,11 mmol) des obigen C-Glycosides mit 39,5 mg (0,14 mmol) 4-Octyloxyphenylboronsäure gemäß Vorschrift 2.
Ausbeute: 28,4 mg (49 %), weißer Feststoff, $[\alpha]_D^{20}$ = +26,8 (c = 0,5, $CHCl_3$).
Phasenverhalten: K 71,5 Ch 118,6 I

Beispiel 26

(1S,6R,8R)-3-(4'-dodecoxyphenyl)-8-(2"-fluor-4"-hexoxyphenyl)-2,4,7-trioxa-3-borabicyclo[4.4.0.]decan

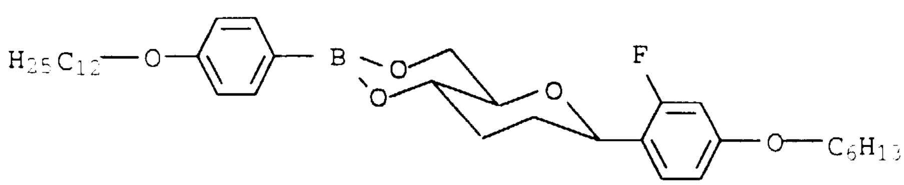

Die Darstellung erfolgt gemäß Beispiel 25. Es wurden 27 mg (0,083 mmol) des C-Glycosides aus Beispiel 25 mit 40,4 mg (0,12 mmol) 4-Dodecyloxyphenylboronsäure umgesetzt.
Ausbeute: 24,8 mg (50 %), weißer Feststoff, $[\alpha]_D^{20}$ = +25,0 (c = 0,2, $CHCl_3$).
Phasenverhalten: K 71,6 Ch 132,5 I

Beispiel 27

(1S, 6R,8R)-3-(3'-fluor-4'-hexoxyphenyl)-8-(2"-fluor-4"-hexoxyphenyl)-2,4,7-trioxa-3-borabicyclo[4.4.0-]decan

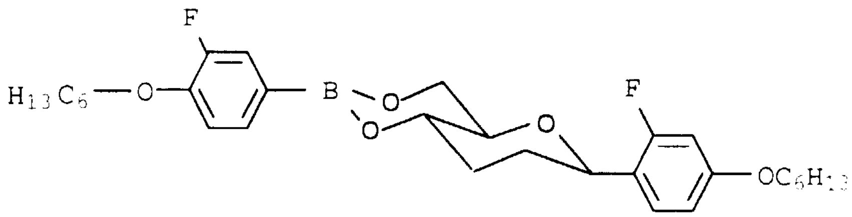

Synthese von 3-Fluor-4-hexoxyphenylboronsäure

Zu einer Lösung von 3 g (10,9 mmol) 4-Brom-2-fluor-hexoxybenzol in 20 ml absolutem Tetrahydrofuran werden unter einer Stickstoffatmosphäre und ständigem Rühren bei -78°C tropfenweise 6,7 ml einer 15%igen Lösung von n-Butyllithium in Hexan (10,9 mmol) zugegeben. Nach 2,5 Stunden Rühren bei -78°C wird der Reaktionsansatz vorsichtig mit 2,4 ml (21,8 mmol) Trimethylborat versetzt. Den Ansatz läßt man über Nacht rühren und dabei bis zur Raumtemperatur aufwärmen.

Am nächsten Tag wird der Ansatz mit 9,9 ml 10%igerSalzsäure versetzt und für eine weitere Stunde bei Raumtemperatur gerührt. Anschließend wird die organische Phase abgetrennt, die wäßrige zweimal mit ca. 50 ml Diethylether ausgeschüttelt, die vereinigten organischen Phasen einmal mit Wasser gewaschen und dann mit Magnesiumsulfat getrocknet. Das Lösungsmittel wird am Rotationsverdampfer abgezogen und der Rückstand aus einem Ethanol-Wasser-Gemisch umkristallisiert.

Ausbeute: 1,07 g (41 %), weiße Kristalle.

Zur Herstellung der Verbindung der obigen Formel wurden 28 mg (0,086 mmol) des C-Glycosides aus Beispiel 25 mit 44,3 mg (0,185 mmol) 3-Fluor-4-hexoxy-phenylboronsäure umgesetzt.

Ausbeute: 13,4 mg (29 %), weißer Feststoff, $[\alpha]_D^{20}$ = +26,0 (c = 0,1, $CHCl_3$).

Phasenverhalten:K 65,3 Ch 118,0 I

Beispiel 28

(1S,6R,8R)-3-(3'-fluor-4'-hexoxyphenyl)-8-(4"-octoxyphenyl)-2,4,7-trioxa-3-borabicyclo[4.4.0.]decan

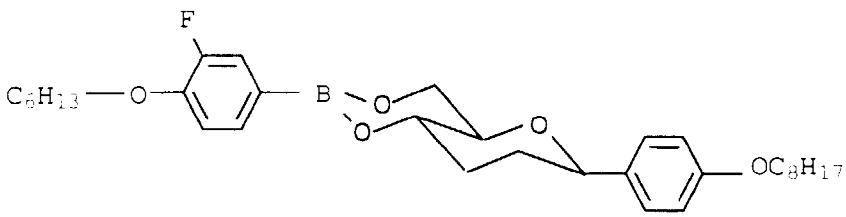

Die Herstellung dieser Verbindung erfolgte nach der allgemeinen Vorschrift 2. Es wurden 30 mg (0,085 mmol) 1-(2,3-Didesoxy-β-D-erythro-hexapyranosyl)-4'-octoxybenzol mit 43 mg (0,179 mmol) der in Beispiel 27 angegebenen Boronsäure umgesetzt.

Ausbeute: 29,0 mg (60 %), weißer Feststoff, $[\alpha]_D^{20}$ = +19,4 (c = 0,5, $CHCl_3$).

Phasenverhalten: K 66,7 ($S_C$* 52,4) $S_A$ 163,7 I.

Beispiel 29

(1S,6R,8R)-3-(3'-fluor-4'-hexoxyphenyl)-8-(4''-decoxyphenyl)-2,4,7-trioxa-3-borabicyclo[4.4.0.]decan

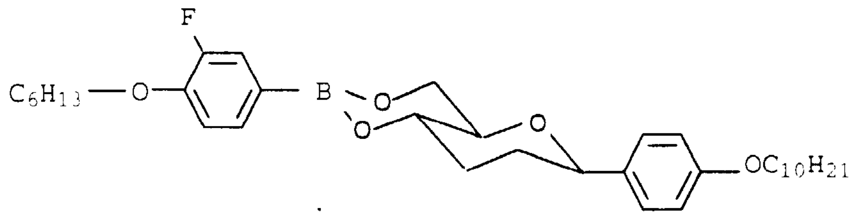

Die Herstellung erfolgte gemäß Beispiel 28. Es wurden 30 mg (0,078 mmol) 1'-(2,3-Didesoxy-β-D-erythro-hexapyranosyl)-4-decyloxybenzol mit 39,5 mg (0,164 mmol) der in Beispiel 27 angegebenen Boronsäure umgesetzt.
Ausbeute: 23,5 mg (50 %), weißer Feststoff, $[\alpha]_D^{20}$ = +17,5 (c = 0,2, $CHCl_3$).
Phasenverhalten: K 67,7 ($S_C$* 39,3) $S_A$ 158,7 I.

Beispiel 30

(1S,6R,8R)-3-(3'-fluor-4'-hexoxyphenyl)-8-(4''-dodecoxyphenyl)-2,4,7-trioxa-3-borabicyclo[4.4.0.]decan

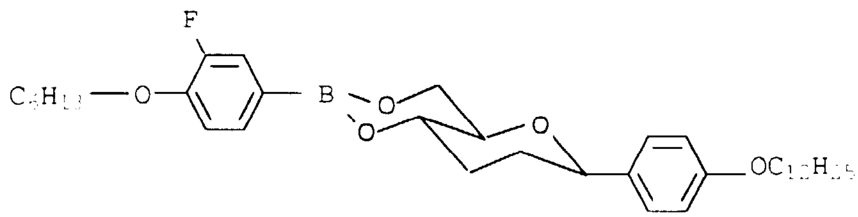

Die Herstellung erfolgte gemäß Beispiel 28. Es wurden 32 mg (0,078 mmol) 1-(2,3-Didesoxy-β-D-erythro-hexapyranosyl)-4-dodecyloxybenzol mit 50 mg (0,208 mmol) der in Beispiel 27 angegebenen Boronsäure umgesetzt.
Ausbeute: 31,5 mg (65 %), weißer Feststoff, $[\alpha]_D^{20}$ = +15,8 (c = 0,5, $CHCl_3$).
Schmelzpunkt: 69,0°C.

Beispiel 31

(1S,6R,8R)-3-(3-fluor-4'-hexoxyphenyl)-8-(4''-tetradecoxyphenyl)-2,4,7-trioxa-3-borabicyclo[4.4.0.]decan

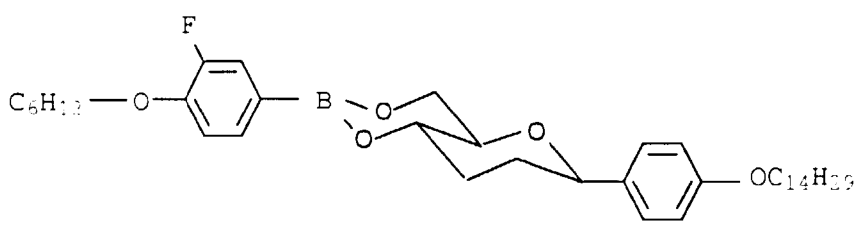

Die Herstellung erfolgte gemäß Beispiel 76. Es wurden 32 mg (0,073 mmol) 1-(2,3-Didesoxy-β-D-erythro-hexapyranosyl)-4-tetradecyloxybenzol mit 50 mg (0,208 mmol) der in Beispiel 27 angegebenen Boronsäure umgesetzt.
Ausbeute: 32,2 mg (67 %), weißer Feststoff, $[\alpha]_D^{20}$ = +16,8 (c = 0,5, $CHCl_3$).
Phasenverhalten: K 73,4 ($S_C$* 28,0) $S_A$ 150,7 I.

**Patentansprüche**

1. Chirale Verbindungen der allgemeinen Formel I

$$A\text{-}Y\text{-}(M\text{-}Y)_n\text{-}z\text{-}(Y\text{-}M)_n\text{-}Y\text{-}A$$

in der

Z ein Rest der Formel

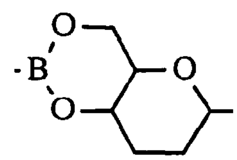

oder

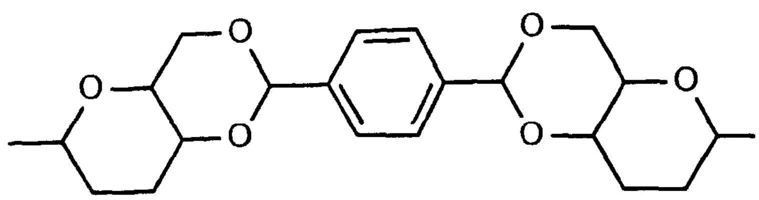

ist und wobei die Reste

A unabhängig voneinander gegebenenfalls durch Fluor, Chlor, Brom oder Cyan substituiertes und gegebenenfalls durch O, S, NH, $NCH_3$, COO oder OCO unterbrochenes $C_1$ bis $C_{20}$-Alkyl,

Y unabhängig voneinander eine chemische Bindung, O, S, NH, $N(CH_3)$, COO oder OCO und

M unabhängig voneinander eine mesogene Gruppe aus einem aromatischen oder aliphatischen Ringsystem der allgemeinen Formel II

$$-Y^1\text{-}(Z^1\text{-}Y^1)_m\text{-} \qquad \text{II}$$

bedeuten, in dem die Reste

$Y^1$ unabhängig voneinander eine direkte Bindung, O, COO, OCO, $CH_2O$, $OCH_2$, CH=N oder N=CH,

$Z^1$ unabhängig voneinander gegebenenfalls durch Fluor, Chlor, Brom, Jod, Cyan, Hydroxy, Methoxy oder Methyl substituiertes p-Phenylen, 1,3,4-Thiadiazolylen-2,5 oder Pyrimidylen-2,5 und

m eine ganze Zahl zwischen 1 und 3 bedeuten,
und

n unabhängig voneinander 0 oder 1 ist mit der Maßgabe, daß ein n von 0 verschieden ist.

2. Verbindungen gemäß Anspruch 1, bei denen die Reste A unabhängig voneinander gegebenenfalls durch O, COO oder OCO unterbrochenes $C_1$- bis $C_{12}$-Alkyl sind.

3. Verbindungen gemäß Anspruch 1, bei denen die Reste Y unabhängig voneinander eine direkte Bindung, O, COO oder OCO sind.

4. Verbindungen gemäß Anspruch 1, bei denen die Reste

A unabhängig voneinander gegebenenfalls durch O, COO oder OCO unterbrochenes $C_1$ bis $C_{12}$-Alkyl und

Y unabhängig voneinander eine direkte Bindung oder O, COO oder OCO sind.

5. Verwendung der Verbindungen gemäß Anspruch 1 als Dotierstoff in flüssigkristallinen Medien.

6. Verwendung von flüssigkristallinen Medien enthaltend Verbindungen gemäß Anspruch 1 in der Displaytechnologie, in optischen, elektronischen sowie opto-elektronischen Speichermedien, der elektronischen Datenverarbeitung und -vervielfältigung oder der Elektrophotografie sowie in Licht reflektierenden Schichten.

## Claims

1. A chiral compound of the general formula I

$$A\text{-}Y\text{-}(M\text{-}Y)_n\text{-}Z\text{-}(Y\text{-}M)_n\text{-}Y\text{-}A$$

where

Z is a radical of the formula

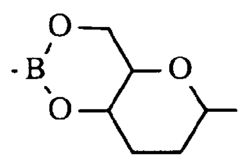

or

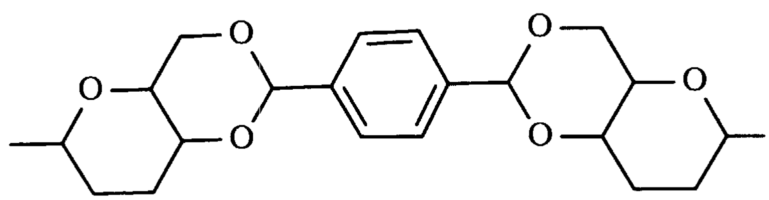

and where the radicals

A independently of one another are $C_1$ to $C_{20}$-alkyl which may be substituted by fluorine, chlorine, bromine or cyano and may be interrupted by O, S, NH, $NCH_3$, COO or OCO,

Y independently of one another are a chemical bond, O, S, NH, $N(CH_3)$, COO or OCO and

M independently of one another are a mesogenic group comprising an aromatic or aliphatic ring system of the general formula II

$$-Y^1-(Z^1-Y^1)_m- \quad \text{II}$$

where the radicals

$Y^1$ independently of one another are a direct bond, O, COO, OCO, $CH_2O$, $OCH_2$, CH=N or N=CH,

$Z^1$ independently of one another are p-phenylene, 1,3,4-thiadiazol-2,5-ylene or 2,5-pyrimidylene, each of which may be substituted by fluorine, chlorine, bromine, iodine, cyano, hydroxyl, methoxy or methyl, and

m is an integer from 1 to 3, and

n independently of one another are 0 or 1 with the proviso that one n is other than 0.

**2.** A compound as claimed in claim 1, where the radicals A independently of one another are $C_1$ to $C_{12}$-alkyl which may be interrupted by O, COO or OCO.

**3.** A compound as claimed in claim 1, where the radicals Y independently of one another are a direct bond, O, COO or OCO.

**4.** A compound as claimed in claim 1, where the radicals

A independently of one another are $C_1$ to $C_{12}$-alkyl which may be interrupted by O, COO or OCO and

Y independently of one another are a direct bond or O, COO or OCO.

**5.** The use of the compounds as claimed in claim 1 as a dope in liquid-crystalline media.

**6.** The use of liquid-crystalline media containing compounds as claimed in claim 1 in display technology, in optical, electronic and optoelectronic storage media, electronic data processing and data duplication or electrophotography and also in light-reflecting layers.

**Revendications**

**1.** Composés chiraux de formule générale I

$$A\text{-}Y\text{-}(M\text{-}Y)_n\text{-}Z\text{-}(Y\text{-}M)_n\text{-}Y\text{-}A$$

dans laquelle

Z est un reste de formule

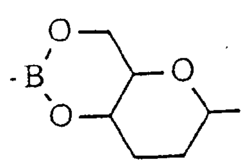

ou

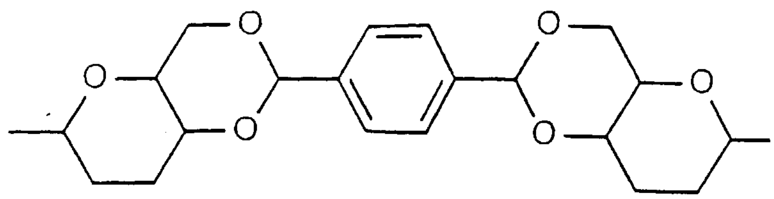

et dans lesquelles les restes

A sont mis, indépendamment les uns des autres, pour un groupement alkyle en $C_1$ - $C_{20}$, éventuellement substitué par un atome de fluor, de chlore, de brome ou par un groupement cyano, et éventuellement interrompu par O, S, NH, $NCH_3$, COO ou OCO,

Y sont mis, indépendamment les uns des autres, pour une liaison chimique, O, S, NH, $N(CH_3)$ COO ou OCO et

M sont mis, indépendamment les uns des autres, pour un groupement mésogène constitué d'un système cyclique aromatique ou aliphatique de formule générale II

$$-Y^1-(Z^1-Y^1)_m- \qquad \text{II}$$

dans laquelle les restes

$Y^1$ sont mis, indépendamment les uns des autres pour une liaison directe, O, COO, OCO, $CH_2O$, $OCH_2$, CH=N ou N=CH,

$Z^1$ sont mis, indépendamment les uns des autres pour un groupement p-phénylène, 1,3,4-thiadiazolylène-2,5 ou pyrimidylène-2,5, éventuellement substitués par un atome de fluor, de chlore, de brome, d'iode, un groupement cyano, hydroxy, méthoxy ou méthyle, et

m est un nombre entier allant de 1 à 3,
et

n valent, indépendamment les uns des autres, 0 ou 1, étant spécifié que l'un des n est différent de 0.

**2.** Composés selon la revendication 1, dans lesquels les restes A sont, indépendamment les uns des autres, des groupements alkyle en $C_1$ - $C_{12}$ éventuellement interrompus par O, COO ou OCO.

**3.** Composés selon la revendication 1, dans lesquels les restes Y sont, indépendamment les uns des autres, une liaison directe, O, COO ou OCO.

**4.** Composés selon la revendication 1, dans lesquels les restes

A sont indépendamment les uns des autres, des groupements alkyle en $C_1$-$C_{12}$ éventuellement interrompus par O, COO ou OCO et

Y sont mis, indépendamment les uns des autres, pour une liaison directe ou O, COO ou OCO.

**5.** Utilisation de composés selon la revendication 1, en tant qu'agent de dopage pour des cristaux liquides.

**6.** Utilisation de cristaux liquides contenant des composés selon la revendication 1 dans le domaine de l'affichage, dans des supports d'enregistrement optiques, électroniques ou opto-électroniques, dans le domaine du traitement de données électronique et de la reproduction ou en électrophotographie ou dans des couches réfléchissant la lumière.